# EUROPEAN PATENT APPLICATION

(11) **EP 3 513 751 A1**
(43) Date of publication of application: **24.07.2019**
(21) Application number: 19152120.2
(22) Date of filing: 16.01.2019
(51) Int. Cl.: A61B 17/34

(54) **SURGICAL ACCESS DEVICE WITH INTEGRAL FILTER AND EVACUATION PORT**

(30) Priority: 17.01.2018 US 201862618138 P; 10.12.2018 US 201816214269
(71) Applicant: Covidien LP, Mansfield, MA 02048 (US)
(72) Inventor: MADDUR, Jeevan Shankar Setty, 560096 Bangalore (IN); VIKHARANKAR, Yogesh Kishor, 500050 Hyderabad (IN)
(74) Representative: Maschio, Antonio

(57) **Abstract**

A seal assembly for use with an access apparatus includes an integral filter and evacuation port for filtering of fluids, e.g., smoke, from an operating site and removing contaminants and/or odor from the fluids for release of the filtered fluids into the ambient atmosphere. The seal assembly may be a separate subassembly or component which is releasably couplable to the access apparatus or may be integral with the access apparatus. The seal assembly defines its own flow path independent of the insufflation mechanism of the cannula assembly, and is capable of filtering fluids even in the presence of a surgical object positioned within the cannula assembly.

## Description

### BACKGROUND

### 1. Technical Field

The present disclosure relates to an access apparatus for accessing a body cavity. More particularly, the present disclosure relates to an assembly for use with an access apparatus and having a filter for filtering and evacuating smoke and other contaminants generated during performance of a surgical procedure.

### 2. Background of Related Art

Minimally invasive surgical procedures including both endoscopic and laparoscopic procedures permit surgery to be performed on organs, tissues and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula accessing the abdominal cavity to perform one or more surgical tasks. The cannula may incorporate a seal to establish a substantially fluid tight seal about the instrument to preserve the integrity of the pneumoperitoneum.

Instruments utilized during a laparoscopic procedure may include lasers, electro-cautery or sonic cutting instruments, which produce smoke and/or an aerosol as a byproduct of treating tissue. Smoke plumes can obscure the clinician's field of vision and the odor generated is unpleasant. Further, the smoke plume may contain infectious agents which may contaminate the operating arena thereby presenting a danger to operating personnel. Chemical vapor may be irritating to the respiratory tract and also may be carcinogenic. The smoke, noxious fumes, and other gases and vapors can include particulates, bacteria, viral elements and undesirable odors.

Conventional methodologies for evacuating smoke include using a surgical smoke evacuation device. This device includes a vacuum pump, tubing, and a filter to filter out particulates and microbials and properly dispose of them. A tube is typically attached to the insufflation port of an access cannula and the smoke is ventilated through the filter. However, this arrangement interrupts the surgical procedure requiring the additional steps of disconnecting the insufflation port from the gas source, mounting the filter to the insufflation port and thereafter reconnecting the gas source to reestablish the pneumoperitoneum to continue the surgical procedure. The separate filter also adds an additional component and expense thereby increasing the cost of the underlying procedure.

Removing the smoke, gases and vapors is typically done through a mechanical filtration method. Because the surgical field is a high moisture environment, the filter tends to clog. The clogged filter and reduced flow rate becomes a limiting factor. Also, it is desirable not to disadvantageously impact pneumoperitoneum.

It would be desirable to provide smoke evacuation during surgery in a compact, efficient arrangement that can also reduce cost.

### SUMMARY

Accordingly, the present disclosure is directed to an assembly for use with an access apparatus to provide filtering of fluids, e.g., smoke, from an operating site and to remove contaminants and/or odor from the fluids for release of the filtered fluids into the ambient atmosphere.

In an aspect of the present disclosure, a surgical access assembly comprises a seal housing and at least one seal for providing a seal around a surgical instrument; a cannula assembly including a stopcock; and a filter assembly attached to the stopcock, the stopcock having an open and a closed position, insufflation gas flowing through the filter assembly when the stopcock is in an open position.

The surgical access assembly can include the filter assembly having a membrane filter. The filter assembly can have a filter female connector and a filter male connector, and the membrane filter can be disposed between the filter female connector and the filter male connector.

The membrane filter can be selected from a group consisting of polyvinylchloride polymer and poly-tetrafluorethylene polymer. The filter assembly can include an additional filter element. The filter assembly can be removably and replaceably attached to the stopcock.

The filter assembly can be disposed downstream of the stopcock. The filter assembly can be disposed upstream of the stopcock.

The stopcock can have a movable member with at least an open and a closed position.

### BRIEF DESCRIPTION OF THE DRAWINGS

Various aspects and features of the present disclosure are described hereinbelow with references to the drawings, wherein:
FIGS. 1-2 are perspective views of a surgical access device having a seal assembly attached thereto;
FIG. 3 is an exploded perspective view of the access device of FIGS. 1-2, illustrating the obturator assembly removed with the seal assembly mounted to the cannula assembly;
FIG. 4 is a side elevation view of an example of a surgical access device; and
FIG. 5 is a side elevation view of the access device of FIG. 4, with a filter assembly shown with parts separated.

### DETAILED DESCRIPTION

Particular embodiments of the present disclosure are described hereinbelow with reference to the accompanying drawings. However, it is to be understood that the disclosed embodiments are merely examples of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to employ the present disclosure in virtually any appropriately detailed structure.

The present disclosure relates to a surgical access device with a filter for filtering out contaminants, smoke, and the like from insufflation gases used during laparoscopic, endoscopic, or other minimally invasive surgery. The filter may be incorporated with an access apparatus, such as a cannula assembly, for the removal and/or treatment of fluids from, e.g., the peritoneal cavity, during a laparoscopic procedure. Such fluids may include smoke and other gaseous material in addition to aerosol and particle byproducts of the laparoscopic procedure involving cutting, heating or burning, and may include, for example, chemicals, ultrasonic vapors, particles, and ion dust particles. More particularly, the present disclosure relates to a surgical access device with a filter assembly and evacuation port that efficiently removes smoke, odor, vapor, particles or plumes released by chemicals or produced by the use of lasers, sonic cutting and/or cautery or other surgical techniques or instruments, (hereinafter, collectively referred to as "contaminated fluids"), from within the peritoneal cavity.

The trocar apparatus has an obturator assembly positionable within a cannula assembly. A filter can be attached to the seal assembly, or incorporated within it. However, the filter can be utilized in other capacities such as, e.g., in hand access systems where the surgeon's hand is introduced within the peritoneal cavity to assist in performing the laparoscopic procedure. The filter may be contemplated for use in surgical procedures in other areas of the body, e.g., in other endoscopic procedures, or other minimally invasive procedures, including arthroscopic, gynecological, spinal procedures, and the like.

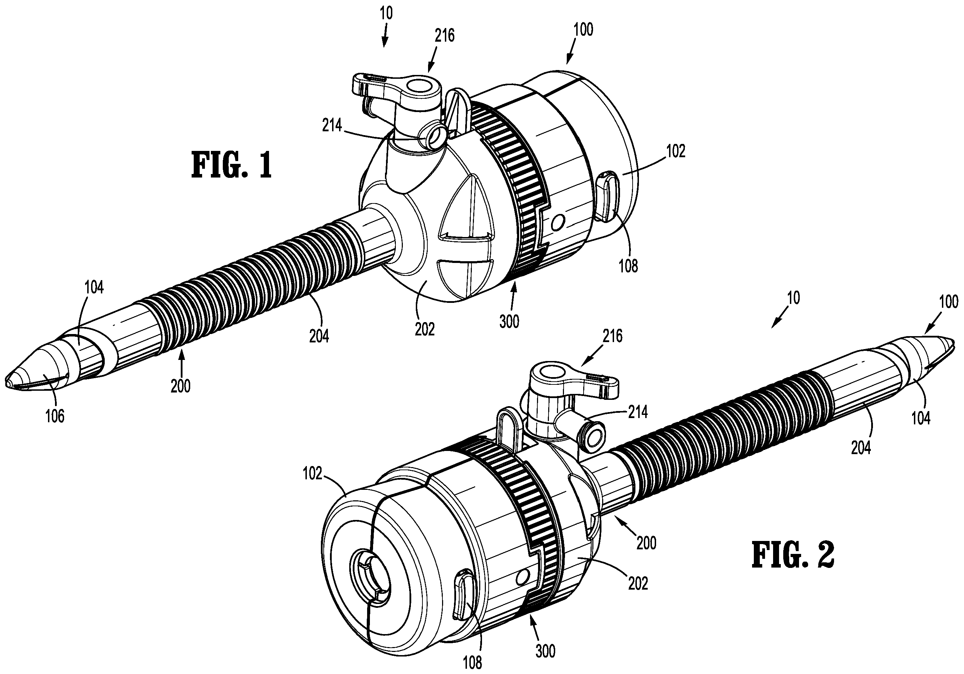

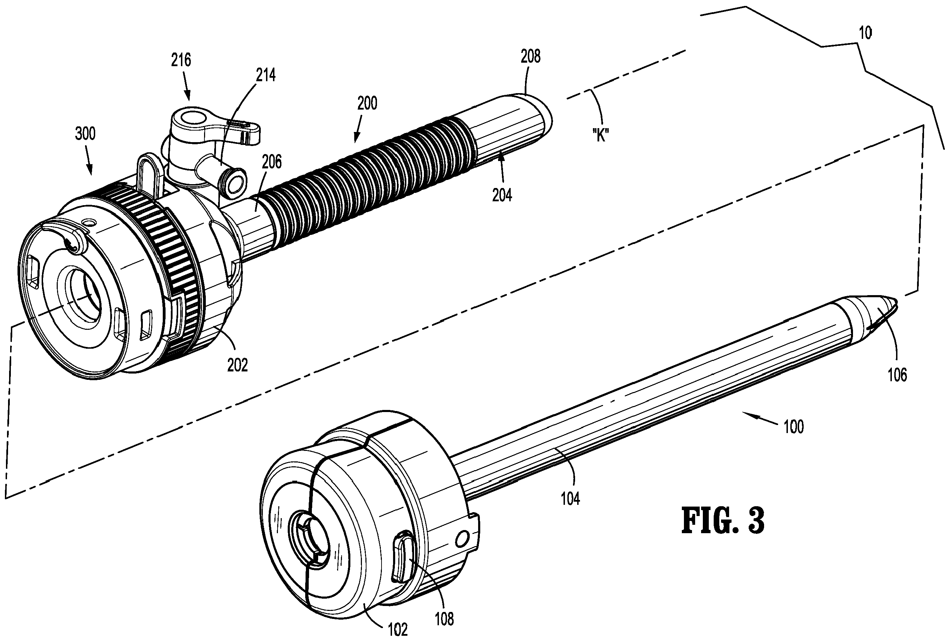

Referring initially to FIGS. 1-3, there is illustrated an access assembly incorporating a seal assembly. The access assembly is intended to permit access to an insufflated peritoneal cavity during a laparoscopic procedure to permit the introduction of a surgical object for performing various surgical tasks on internal organs within the cavity. The surgical object may be a surgical instrument such as laparoscopic or endoscopic clip appliers, graspers, dissectors, retractors, staplers, laser probes, photographic devices, tubes, endoscopes and laparoscopes, electro-surgical devices, and the like.

In an example of an access apparatus, a trocar apparatus 10, which includes an obturator assembly 100, a cannula assembly 200 for at least partial reception of the obturator assembly 100, and a seal assembly 300 which is selectively mountable to the cannula assembly 200 to provide sealing capabilities, e.g., to establish a sealing relation about an inserted surgical object. In general, the obturator assembly 100 includes an obturator handle 102 and an elongated obturator member 104 extending from the obturator handle 102. The obturator member 104 typically includes a penetrating end 106 for passage through tissue. In some embodiments, the penetrating end 106 is closed and transparent to permit visualization during entry of the trocar apparatus 10 within an insufflated body cavity such as the peritoneal cavity, e.g., with an endoscope introduced through the obturator assembly 100. The obturator assembly 100 may include a mechanism to permit selective coupling with either or both the cannula assembly 200 and with the seal assembly 300 such as a pair of latches 108 which engage corresponding structure of the assemblies 200, 300.

The cannula assembly 200 includes a cannula housing 202 and a cannula sleeve 204 extending from the cannula housing 202. The cannula sleeve 204 defines proximal and distal ends 206, 208 and a longitudinal axis "k" extending along the length of the cannula sleeve 204. The cannula housing 202 and the cannula sleeve 204 define a longitudinal opening (not shown) for reception and passage of the surgical object. The cannula housing 202 may include a zero closure valve, e.g., a duckbill valve, which is configured to close in the absence of a surgical object to prevent egress of insufflation gases. The zero closure valve does not typically establish a seal about an inserted surgical object. The cannula housing 202 also includes an insufflation port 214 and associated insufflation valve 216 (e.g., a stop cock valve) for selective introduction of insufflation fluids into the cannula sleeve 204 and the peritoneal cavity. Further details of an obturator assembly 100 and cannula assembly 200 for use with the seal assembly 300 may be ascertained by reference to commonly assigned U.S. Publication No. 2015-0216560 to Holsten, the entire contents of which is hereby incorporated by reference herein.

The seal assembly 300 will be discussed. The seal assembly 300 is selectively, e.g., releasably, couplable to the cannula housing 202 of the cannula assembly 200 to provide sealing capabilities about an inserted surgical object. Any mechanism for releasably mounting the seal assembly 300 to the cannula housing 202 is envisioned including, e.g., a friction fit, bayonet coupling, snap fit, and the like. The seal assembly 300 includes a seal housing defining a seal axis which is in general longitudinal alignment with the longitudinal axis "k" of the cannula sleeve 204 in the assembled condition of the components. The seal housing may include one or more housings.

In the example shown in FIGS. 1-3, the seal assembly 300 further includes a filter (not shown) disposed within the housing component or components and an object seal. The filter is disposed proximal of the object seal. The seal assembly 300 also includes an evacuation port and an evacuation valve adjacent the evacuation port for selective release of the fluids from the peritoneal cavity. In other examples, the filter can be proximal the zero closure (duckbill) seal, or located elsewhere on the cannula assembly. The outer surface of the housing component or components may include roughened surfaces, e.g., ribs, scallops, and the like, to facilitate engagement and manipulation by the user.

The filter may be an ultra-high molecular filter 310 (or ULPA filter), activated carbon filter, a high efficiency particulate air filter (or HEPA filter) 310, or a combination of two or more of them. In an example, the filter 310 includes a high-density polyethylene material (HDPE) or a polyurethane material, with activated charcoal. The filter desirably has a plurality of turns, pleats, and/or layers. In this example, the filter is capable of providing a flow rate of at least 6 liters per minute (at 15 mm mercury) from the peritoneal cavity through the filter 310 and into the ambient environment. The filter can be capable of removing smoke and contaminant particles from the fluid including nanoparticles or ultrafine particles of less than 0.12 microns in diameter with an efficiency rate of least 99.995%. These particles may be responsible for causing systemic diseases as a result of chronic exposure in operating rooms to health care personnel. Other filter arrangements are contemplated. The filter 310 may be an ultra-low particulate air filter (ULPA filter) with or without carbon or other odor reducing elements. The filter can include a combination of film, resins and/or activated carbon. The filter can be incorporated into the seal housing, or be part of a separate, attachable component.

In one example, a glass filter material incorporating a carbon material was used. That material was found to be hydrophilic. A polytetra-fluoroethylene ("PTFE") material incorporating carbon was also used. That material was found to be hydrophobic. Since the surgical field is a very wet environment, the hydrophobic material was preferred, as the hydrophobic material tended to clog less.

The filter may be made from carbon incorporated into a polymer resin, granular carbon incorporated in a sheet, a fabric that was a spun-fiber material impregnated with carbon, or any other appropriate material.

In further examples, the filter can be a material incorporating carbon, and including an ULPA material. For example, the carbon material can be molded into a shape, and also have a layer of ULPA material on the top, the bottom, both the top and the bottom, or otherwise disposed adjacent the carbon filter element. In addition, the filter can be made from ULPA filter material that comes in a sheet, and is shaped and folded so as to have folds or pleats. In addition, that filter element can have a layer of carbon material on top, on the bottom, on both the top and bottom, or otherwise disposed alongside the ULPA filter element.

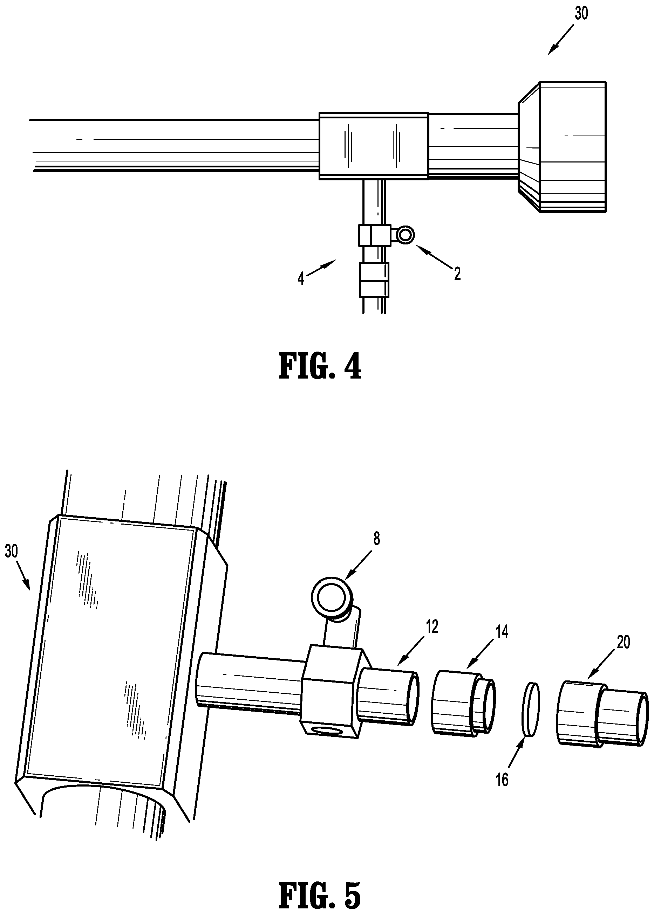

It is a common practice to evacuate contaminated surgical smoke by opening the stopcock on one of the trocars and letting the insufflation gas carry the smoke into the operating room. However, smoke, noxious fumes, and other gases and vapors can include particulates, bacteria, viral elements and undesirable odors. The escaping air is desirably filtered and cleared of these contaminants. In an example shown in FIGS. 4 and 5, an access device in the form of a trocar 30 is shown with the obturator removed. A removable filter assembly 4 is attached to the stopcock 2 of the trocar 30. An enlarged view of the filter assembly 4 and its attachment to the stopcock 2 is shown in FIG. 5. The filter assembly 4 has a filter female connector 14, which connects to the trocar luer 12, a membrane filterl6, and a filter male connector. The filter assembly can be partially or entirely disposable. In this example, the filter can be made of medical grade polymers, such as polyvinylchloride ("PVC") polymer or poly-tetrafluoroethylene ("PTFE") polymer. For example, GE Whatman PM2.5 PTFE membrane filters or Merck's PVC membrane filters can be used.

The filter assembly can be removable and replaceable, or incorporated permanently with the trocar. The filter assembly is part of a flow path of insufflation gas through the cannula of the cannula assembly that extends through the stopcock. The stopcock has a movable member (best seen in FIGS. 1-3) that is movable between at least an open and a closed position. The filter assembly is disposed as downstream the stopcock 8as shown in FIGS. 4 and 5, or it can be disposed upstream the stopcock 8, or elsewhere on the cannula assembly.

The filter assembly can have one such membrane filter, or include additional filter elements such as those discussed above. In use, one or more of the trocars being used in the procedure may incorporate a filter assembly like filter assembly 4. It is also contemplated that the trocar 30 can be designed with a stopcock 2 that is larger or of a different design to accommodate the filter assembly 4 and maximize the gas flow through the stopcock 2. It is also contemplated that the disposable filter assembly 4 can be used with a trocar 30 that can, in whole or in part, be re-sterilized and re-used. Furthermore, the filter assembly can be made partially re-usable. For example, the filter connectors 14 and 20 can be re-usable and the filter 16 can be a removable and replaceable part or assembly. The benefits of the filter assembly include ease of use, minor changes to a typical trocar, and inexpensiveness.

A method of performing surgery is contemplated in which a plurality of trocars are inserted into a patient's body. One or more of the plurality of trocars has a removable filter assembly. During the surgical procedure, the stopcock is opened to allow insufflation gas to flow therethrough, on one or more of the trocars. The method may include removing a filter assembly or filter assemblies from a trocar or trocars, sterilizing the trocar, and attaching a new filter assembly. It is contemplated that a drop in gas flow through the filter can be utilized to indicate that the filter needs replacement. This can be observed directly, or the device can include sensors and/or a visual or audible indication system.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A surgical access assembly comprising: a seal housing and at least one seal for providing a seal around a surgical instrument; a cannula assembly including a stopcock; and a filter assembly attached to the stopcock, the stopcock having an open and a closed position, insufflation gas flowing through the filter assembly when the stopcock is in an open position.
2. The surgical access assembly according to paragraph 1, wherein the filter assembly has a membrane filter.
3. The surgical access assembly according to paragraph 2, wherein the filter assembly has a filter female connector and a filter male connector, and the membrane filter is disposed between the filter female connector and the filter male connector.
4. The surgical access assembly according to paragraph 2, wherein the membrane filter is selected from a group consisting of polyvinylchloride polymer and poly-tetrafluorethylene polymer.
5. The surgical access assembly according to paragraph 2, wherein the filter assembly includes an additional filter element.
6. The surgical access assembly according to paragraph 1, wherein the filter assembly is removably and replaceably attached to the stopcock.
7. The surgical access assembly according to paragraph 1, wherein the filter assembly is disposed downstream of the stopcock.
8. The surgical access assembly according to paragraph 1, wherein the filter assembly is disposed upstream of the stopcock.
9. The surgical access assembly according to paragraph 1, wherein the stopcock has a movable member with at least an open and a closed position.

## Claims

1. A surgical access assembly comprising: a seal housing and at least one seal for providing a seal around a surgical instrument; a cannula assembly including a stopcock; and a filter assembly attached to the stopcock, the stopcock having an open and a closed position, insufflation gas flowing through the filter assembly when the stopcock is in an open position.

2. The surgical access assembly according to claim 1, wherein the filter assembly has a membrane filter.

3. The surgical access assembly according to claim 2, wherein the filter assembly has a filter female connector and a filter male connector, and the membrane filter is disposed between the filter female connector and the filter male connector.

4. The surgical access assembly according to any preceding claim, wherein the membrane filter is selected from a group consisting of polyvinylchloride polymer and poly-tetrafluorethylene polymer.

5. The surgical access assembly according to any preceding claim, wherein the filter assembly includes an additional filter element.

6. The surgical access assembly according to any preceding claim, wherein the filter assembly is removably and replaceably attached to the stopcock.

7. The surgical access assembly according to any of claims 1 to 5, wherein the filter assembly is disposed downstream of the stopcock.

8. The surgical access assembly according to any of claims 1 to 5, wherein the filter assembly is disposed upstream of the stopcock.

9. The surgical access assembly according to any preceding claim, wherein the stopcock has a movable member with at least an open and a closed position.
